# FASCICULE DE BREVET EUROPEEN

(11) **EP 2 747 582 B1**
(45) Date de publication et mention de la délivrance du brevet: **21.12.2016**
(21) Numéro de dépôt: 12743467.8
(22) Date de dépôt: 07.08.2012
(51) Int. Cl.: A23L 27/20, A23P 10/20, C07C 47/58

(54) **PROCEDE DE PREPARATION D'UNE COMPOSITION AROMATIQUE COMPRENANT UN COMPOSE A BASE DE DEUX SOLIDES PRESENTANT DES PROPRIETES ORGANOLEPTIQUES**
HERSTELLUNGSVERFAHREN FÜR EINE AROMAZUSAMMENSETZUNG ENTHALTEND EINE VERBINDUNG BASIEREND AUF ZWEI FESTSTOFFEN MIT ORGANOLEPTISCHEN EIGENSCHAFTEN
PROCESS FOR PREPARING A FLAVOUR COMPOSITION COMPRISING A COMPOUND BASED ON TWO SOLIDS PRESENTING ORGANOLEPTIC PROPERTIES

(30) Priorité: 25.08.2011 FR 1157521
(43) Date de publication de la demande: 02.07.2014
(73) Titulaire: Rhodia Operations, 75009 Paris (FR)
(72) Inventeur: LE-THIESSE, Jean-Claude, F-42100 Saint-Etienne (FR); MASSON, Jean-Claude, F-69003 Lyon (FR); COCHENNEC, Corine, F-38500 Voiron (FR); GIACOMONI, Olivier, F-69270 Fontaines sur Saône (FR)
(74) Mandataire: Ridray, Annabelle
(86) Numéro de dépôt international: PCT/EP2012/065468
(87) Numéro de publication internationale: WO 2013/026699

(56) Documents cités:
- EP-A1- 0 077 639
- EP-A1- 0 689 772
- WO-A1-2010/046239
- WO-A1-2011/104208
- FR-A1- 2 482 977
- JP-A- 2008 214 233
- US-A- 5 009 900
- US-A1- 2004 018 954
- DATABASE CAPLUS, [Online] 28 février 1968 (1968-02-28), ZENON RYSZARD ET AL: "Food essence with vanilla flavor", XP002533462, extrait de CAPLUS Database accession no. 1969-402311

## Description

La présente invention se rapporte au domaine de la préparation de compositions pulvérulentes, c'est-à-dire sous forme de poudre, présentant des propriétés organoleptiques. Plus précisément, elle se rapporte à un procédé de préparation d'une composition aromatique pulvérulente comprenant essentiellement un composé à base d'au moins deux solides présentant des propriétés organoleptiques, en particulier à base de vanilline et d'éthylvanilline.

Les produits et compositions aromatiques au sens où elles présentent des propriétés organoleptiques sont utilisés dans de nombreux domaines d'application en tant qu'arôme et/ou parfum. En particulier, ces produits et compositions aux propriétés organoleptiques se trouvent abondamment consommés dans l'industrie alimentaire et animale. Ils trouvent également des applications dans d'autres domaines tels que la pharmacie ou la parfumerie. Il s'ensuit que ce sont des produits et des compositions de grande consommation. Les propriétés organoleptiques d'un produit ou d'une composition à base d'au moins deux produits peuvent être définies comme l'ensemble des caractéristiques perçues et évaluées par les sens du consommateur. Ces propriétés jouent un rôle primordial dans la perception desdits produits et compositions avant usage ou consommation et dans leur appréciation lorsqu'ils sont consommés ou utilisés. Les principaux éléments contribuant à la qualité organoleptique sont notamment l'aspect visuel (forme, couleur...), la consistance ou texture, le goût, l'odeur, les arômes.

Compte tenu de l'intérêt croissant pour les compositions organoleptiques dans l'industrie agro-alimentaire, pharmaceutique, la parfumerie, le besoin pour un procédé simple et aisément exploitable à l'échelle industrielle pour la fabrication de telles compositions organoleptiques est grand. En particulier, les industriels des secteurs concernés recherchent la fabrication de compositions organoleptiques qui se trouvent sous forme pulvérulente à température ambiante. Or la fabrication de telles compositions est peu décrite dans la littérature.

La Demanderesse a toutefois déjà proposé un procédé pour la préparation d'une composition comprenant essentiellement un composé à base de vanilline et d'éthylvanilline dans la demande WO 2010/046239. Ledit procédé comprend la co-cristallisation de vanilline et d'éthylvanilline mises en oeuvre dans un ratio molaire vanilline/éthylvanilline de 2, en milieu fondu ou en solution dans un solvant les solubilisant. Toutefois, ce procédé présente l'inconvénient d'être difficilement transposable à l'échelle industrielle car la cristallisation du composé est assez lente. En effet, ledit composé présente un phénomène de surfusion, c'est-à-dire que lorsque le produit est fondu et qu'il est refroidi au-dessous de son point de fusion, il cristallise difficilement et reste longtemps à l'état liquide. Le temps nécessaire à la cristallisation est plus ou moins aléatoire et il importe de bien maîtriser la cristallisation.

La présente invention se propose donc de pallier ces inconvénients, particulièrement observés dans le cas d'un composé à base de vanilline et d'éthylvanilline. De manière plus générale, elle propose un nouveau procédé de préparation d'une composition pulvérulente à température ambiante présentant des propriétés organoleptiques. Un des objectifs recherchés par la présente invention est la mise à disposition d'un procédé reposant sur un mode opératoire simple à mettre en oeuvre à l'échelle industrielle en visant spécialement à éviter un contrôle très délicat de la température pour la fabrication d'une composition pulvérulente et présentant des propriétés organoleptiques.

Par ailleurs, compte tenu des difficultés industrielles et des pertes financières générées par le mottage des poudres alimentaires, ce phénomène défini comme l'agglomération de particules étant par exemple désormais bien connu dans le cas de la préparation d'un mélange à sec de poudres de vanilline et d'éthylvanilline, un autre objectif recherché par la présente invention est la mise au point d'un procédé réalisé dans des conditions selon lesquelles le mottage n'est pas favorisé de manière à obtenir une composition organoleptique pulvérulente dont la granulométrie satisfait parfaitement les attentes exprimées par les industriels.

La présente invention a pour objet un procédé de préparation d'une composition aromatique pulvérulente présentant une température de fusion Tf comprenant :
i) l'introduction dans un mélangeur dont l'enceinte a été préalablement chauffée à une température T inférieure à Tf d'au moins deux solides élémentaires pulvérulents présentant des propriétés organoleptiques, lesdits solides étant introduits séparément dans ledit mélangeur,
ii) le mélange dans ledit mélangeur, en l'absence de toute phase liquide extérieure, desdits solides à une température T inférieure à la température Tf, au moins l'un desdits solides élémentaires pulvérulents étant introduit dans ledit mélangeur à une température Ti telle que le mélange se réalise en conditions isothermes à une température fixée à ladite température T, et
iii) la récupération de ladite composition aromatique pulvérulente.

Ladite étape i) du procédé de préparation selon l'invention met en oeuvreau moins deux solides élémentaires pulvérulents, c'est-à-dire sous forme de poudre, présentant chacun des propriétés organoleptiques. Ils sont choisis de sorte que la composition aux propriétés organoleptiques préparée selon le procédé de l'invention se trouve sous forme pulvérulente à température ambiante. Selon l'invention, la température ambiante est définie par une plage de température allant généralement de 10 à 30°C, préférentiellement de 15 à 30°C et plus préférentiellement de 15 à 25°C. Plus particulièrement, lesdits solides élémentaires pulvérulents présentent avantageusement une température de fusion supérieure ou égale à 40°C, très avantageusement supérieure ou égale à 50°C. Lesdits solides élémentaires présentent généralement un température de fusion inférieure à 220°C. De manière préférée, on choisit des solides élémentaires dont les températures de fusion sont relativement proches, c'est-à-dire des solides dont l'écart entre les températures de fusion est d'au plus 50°C, de préférence d'au plus 30°C, de manière plus préférée d'au plus 20°C et de manière encore plus préférée d'au plus 10°C.

De manière préférée, lesdits solides présentant chacun des propriétés organoleptiques sont constitués d'une molécule odorante présentant au moins un hétéroatome choisi parmi l'azote, l'oxygène et le soufre. Lesdites molécules peuvent être cycliques ou acycliques, saturées ou insaturées. Ces molécules odorantes, utilisées en tant qu'arôme ou parfum, interviennent dans un mécanisme dans lequel les organes de l'olfaction sont impliqués.

De manière plus préférée, lesdits solides sont choisis parmi les aldéhydes aromatiques, les cétones aromatiques, les acides aromatiques, les esters aromatiques, les éthers aromatiques, les phénols, les hétérocycles oxygénés, les hétérocycles azotés, les hétérocycles soufrés et azotés, les composés soufrés, les terpènes et les muscs.

Parmi les aldéhydes aromatiques, on choisit avantageusement le 4-hydroxybenzaldéhyde, le 4-hydroxy-3-méthoxybenzaldéhyde (vanilline), le 4-hydroxy-3-éthoxybenzaldéhyde (éthylvanilline), le 4-acétoxy-3-méthoxybenzaldéhyde (acétate de vanilline), le 3,4-diméthoxybenzaldéhyde (Veratraldéhyde), le 2-hydroxy-4-méthylbenzaldéhyde (m-homosalicylaldéhyde), le (2E)-3-(4-méthoxyphenyl)propénal (trans-p-méthoxycinnamaldéhyde).

Parmi les cétones aromatiques, on choisit avantageusement le 4-(4-hydroxyphényl) butan- 2-one (frambinone), la benzophénone, la 1-(4-hydroxy-3-méthoxy)éthanone (acétovanillone), la 1-(3,4-diméthoxyphenyl)éthanone (3',4'-diméthoxyacétophénone), la 4-(4-Hydroxy-3-méthoxyphényl)-3-butèn-2-one (vanillalacétone), la 4-méthyl-2H-1,5-benzodioxepin-3(4H)-one (calone).

Parmi les acides aromatiques, on choisit préférentiellement l'acide 4-Hydroxy-3-méthoxybenzoique (acide vanillique), l'acide 2-méthoxybenzoique (acide o-anisique), l'acide 4-méthoxybenzoique (acide para-anisique), l'acide 3-méthoxybenzoique (acide meta-anisique), l'acide 2-hydroxybenzoique (acide salicylique), l'acide 4-hydroxybenzoique (acide para-hydroxybenzoique), l'acide (2E)-3-phényl-2-propenoique (acide trans-cinnamique), l'acide phénoxyacétique, l'acide 3-phénylpropionique (acide hydrocinnamique).

Parmi les esters aromatiques, on choisit préférentiellement le 4-hydroxy-3-méthoxybenzoate de méthyle (vanillate de méthyle), le 2-hydroxybenzoate de 2-phényléthyle (salicylate de phénethyle), le 2-hydroxybenzoate de phényle (salicylate de phényle), le 4-méthoxybenzoate de méthyle (p-anisate de méthyle), l'acétate de 3,4-méthylènedioxybenzyle (acétate d'héliotropyle ou acétate de piperonyle).

Parmi les éthers aromatiques, on choisit préférentiellement le 1,4-diméthoxybenzène (para-diméthoxybenzène, PDMB), le 2-méthoxy-1-(phénylméthoxy)-4-(1-propényl)benzène (benzyl isoeugénol), le 1-éthoxy-2-hydroxy-4-propénylbenzène (propénylguétol).

Parmi les phénols, on choisit préférentiellement le (4-hydroxy-3-méthoxyphényl)méthanol (alcool vanillique), le 4-hydroxyphénylméthanol (alcool 4-hydroxybenzylique), le 1,3-dihydroxybenzène.

Parmi les hétérocycles oxygénés, on choisit préférentiellement la 3-hydroxy-2-méthyl-4-pyrone, la 3-hydroxy-2-méthyl-gamma-pyrone (maltol), la 2-éthyl-3-hydroxy-4H-pyran-4-one (éthyl maltol), la 1,2-benzopyrone (coumarine), la 4-hydroxy-5-méthyl-3(2H)-furanone (59) (norfuraneol), la 4,6-diméthyl-2H-pyran-2-one (levistamel), le (-)-dodécahydrotétraméthyl naphthofurane (ambroxane).

Parmi les héterocycles azotés, on choisit préférentiellement la 2,6-diméthylpyrazine, la 2-acétylpyrazine (acétylpyrazine), la 2,3,5,6-tétraméthylpyrazine (tétraméthylpyrazine), le 1,4-diazabenzène (pyrazine).

Parmi les composés soufrés, on choisit préférentiellement le disulfure de diphényle, le disulfure de dibenzyle, le disulfure de dicyclohexyle.

Parmi les terpenes, on choisit préférentiellement le (1 R,2S,5R)-5-méthyl-2-(propan-2-yl)cyclohexanol ou [(-)-menthol], le (alphaR,1R,2R,4aS,8aS)-alpha-éthenyldécahydro-2-hydroxy-alpha,2,5,5,8a-pentaméthyl-1-naphthalènepropanol ou [(-)-Sclareol], le (1 R,4S,4aS,6R,8aS)-Octahydro-4,8a,9,9-tetramethyl-1,6-methanonaphthalen-1(2H)-ol ou le (-)-alcool de patchouli ou [(-)-Patchoulol].

Parmi les muscs, on choisit préférentiellement des muscs synthétiques tels que le 3,4,6,7,8-hexahydro-4,6,6,7,8,8-hexaméthylcyclopenta(g)-2-benzopyrane (galaxolide), la cyclopentadécanone (exaltone), la 6-acétyl-1,1,2,4,4,7-hexaméthyltétraline (tonalide), l'acétyl-dinitro-butyl-xylène (musc cétone).

Selon un premier mode particulier de mise en oeuvredu procédé selon l'invention, chacun desdits solides élémentaires est choisi parmi une même famille chimique choisie parmi les familles constituées par les aldéhydes aromatiques, les cétones aromatiques, les acides aromatiques, les esters aromatiques, les éthers aromatiques, les phénols, les hétérocycles oxygénés, les hétérocycles azotés, les hétérocycles soufrés et azotés, les composés soufrés, les terpènes et les muscs. Les composés préférés pour la mise en oeuvredudit premier mode particulier et appartenant auxdites familles sont ceux déjà décrits ci-dessus.

Conformément audit premier mode, chacun desdits solides appartient préférentiellement à la famille des aldéhydes aromatiques. De manière préférée, l'un des solides élémentaires est la vanilline et l'autre solide élémentaire est l'éthylvanilline. On rappelle que la vanilline correspond à la molécule de formule 4-hydroxy-3-méthoxybenzaldéhyde et que l'éthylvanilline correspond à la molécule de formule 4-hydroxy-3-éthoxybenzaldéhyde. La vanilline est très souvent associée à l'éthylvanilline car la présence d'une faible quantité d'éthylvanilline permet d'exalter les propriétés parfumantes et/ou organoleptiques de la vanilline. La vanilline et l'éthylvanilline avantageusement mises en oeuvredans le procédé selon l'invention peuvent avoir été produits par toute synthèse chimique, quelque soit le substrat de départ. La vanilline peut encore avoir été produite selon un procédé biochmique, en particulier un procédé de fermentation microbiologique, notamment de l'acide férulique.

Selon un second mode particulier de mise en oeuvre du procédé selon l'invention, chacun desdits solides élémentaires appartient à une famille chimique différente choisie parmi les familles constituées par les aldéhydes aromatiques, les cétones aromatiques, les acides aromatiques, les esters aromatiques, les éthers aromatiques, les phénols, les hétérocycles oxygénés, les hétérocycles azotés, les hétérocycles soufrés et azotés, les composés soufrés, les terpènes et les muscs. Les composés préférés pour la mise en oeuvre dudit second mode particulier et appartenant auxdites familles sont ceux déjà décrits ci-dessus.

Conformément audit second mode, au moins l'un des solides élémentaires appartient préférentiellement à la famille des aldéhydes aromatiques, des éthers aromatiques ou des hétérocyles oxygénés. De manière plus préférée, l'un des solides élémentaires appartient à la famille des aldéhydes aromatiques et l'autre solide élémentaire appartient à la famille des éthers aromatiques. Par exemple, l'un des solides élémentaires est la vanilline et l'autre solide est le propénylguétol. Une autre combinaison avantageuse conforme audit second mode consiste à choisir l'un des solides élémentaires parmi les éthers aromatiques, par exemple le para-diméthoxybenzène, et l'autre solide élémentaire parmi les hétérocyles oxygénés, par exemple la coumarine.

De manière préférée, au moins l'un desdits solides élémentaires pulvérulents est cristallisé et de manière encore plus préférée, lesdits deux solides élémentaires pulvérulents sont cristallisés.

Selon un troisième mode particulier de mise en oeuvre du procédé selon l'invention, ledit procédé est mis en oeuvre en présence d'au moins trois solides élémentaires pulvérulents présentant chacun des propriétés organoleptiques. De manière préférée, chacun desdits trois solides sont choisis parmi les aldéhydes aromatiques, les cétones aromatiques, les acides aromatiques, les esters aromatiques, les éthers aromatiques, les phénols, les hétérocycles oxygénés, les hétérocycles azotés, les hétérocycles soufrés et azotés, les composés soufrés, les terpènes et les muscs.

Conformément à l'invention, lesdits solides élémentaires sont introduits dans le mélangeur selon tout ratio molaire convenant pour la préparation de ladite composition pulvérulente aromatique obtenue selon le procédé de l'invention.

Selon le mode particulier de réalisation du procédé de l'invention consistant à préparer une composition aromatique pulvérulente comprenant essentiellement un composé à base de vanilline et d'éthylvanilline, la vanilline et l'éthylvanilline sont introduites dans ledit mélangeur selon un ratio molaire vanilline/éthylvanilline au moins égal à 2, préférentiellement compris entre 2 et 3 et très préférentiellement entre 2 et 2,6. Selon ce mode particulier de réalisation de l'invention, la vanilline et l'éthylvanilline sont avantageusement introduites dans ledit mélangeur dans les proportions suivantes :
- de 65 à 90 %, de préférence de 65 à 80 %, de manière plus préférée de 65 à 75%, de manière encore plus préférée de 67 à 70% en poids de vanilline,
- de 10 à 35 %, de préférence de 20 à 35 %, de manière plus préférée de 25 à 35%, de manière encore plus préférée de 30 à 33% en poids d'éthylvanilline.

Conformément à ladite étape i) du procédé selon l'invention, lesdits solides élémentaires pulvérulents sont introduits de manière séparée, c'est-à-dire chacun à l'état de corps pur, dans ledit mélangeur. Lesdits solides peuvent être introduits simultanément ou l'un après l'autre dans n'importe quel ordre. Chacun desdits solides peut être introduit progressivement ou non. Préalablement à l'introduction desdits solides, l'enceinte dudit mélangeur est chauffée à une température T inférieure à la température de fusion du solide élémentaire présentant la plus basse température de fusion, ledit solide étant choisi parmi lesdits solides élémentaires mis en oeuvre dans le procédé selon l'invention. Ladite température T est inférieure à la température de début de fusion de la composition aromatique préparée selon le procédé de l'invention. Selon le mode particulier de réalisation du procédé de l'invention consistant à préparer une composition aromatique comprenant essentiellement un composé à base de vanilline et d'éthylvanilline, l'enceinte dudit mélangeur est chauffée à une température T avantageusement comprise entre 48 et 53°C, très avantageusement comprise entre 51 et 52°C.

Lesdites étapes i) et ii) du procédé selon l'invention sont conduites dans un mélangeur. Il s'agit avantageusement d'un mélangeur à socs de charrue, d'un mélangeur à palettes ou d'un mélangeur à ruban(s), ces mélangeurs étant bien connus de l'Homme du métier versé dans le domaine du mélange de poudres. Ledit mélangeur est avantageusement pourvu d'une double enveloppe afin d'assurer les différents transferts thermiques par circulation d'un fluide caloporteur, avantageusement de l'eau, dans la double enveloppe. Le fluide caloporteur est maintenu à une température supérieure ou égale à la température T à l'intérieur de l'enceinte dudit mélangeur. Par exemple, le fluide caloporteur est maintenu à une température supérieure de 1 à 5°C à ladite température T.

Lesdits deux solides élémentaires pulvérulents aux propriétés organoleptiques sont introduits de façon séparée dans ledit mélangeur puis y sont mélangés selon ladite étape ii) du procédé selon l'invention.

Conformément au procédé selon l'invention, au moins l'un desdits solides élémentaires pulvérulents est préalablement chauffé à une température Ti avant d'être introduit dans ledit mélangeur, ladite température Ti étant voisine de ladite température T à laquelle est effectué le mélange selon ladite étape ii). De manière préférée, lesdits deux solides élémentaires pulvérulents sont introduits à une température Ti ; lesdits deux solides peuvent être préalablement chauffés et introduits dans ledit mélangeur à la même température Ti ou à une température Ti légèrement différente. De manière plus précise, ladite température Ti est avantageusement choisie telle que T - 20°C ≤ Ti ≤ T + 20°C. Selon le mode particulier de réalisation du procédé de l'invention consistant à préparer une composition aromatique pulvérulente comprenant essentiellement un composé à base de vanilline et d'éthylvanilline, la vanilline et l'éthylvanlline sont préalablement chauffées à une température comprise entre 50 et 65°C, de préférence à une température comprise entre 55 et 62°C, de manière encore plus préférée entre 58 et 61 °C.

Conformément à ladite étape ii) du procédé selon l'invention, le mélange desdits solides élémentaires pulvérulents présentant des propriétés organoleptiques est réalisé en conditions isothermes, c'est-à-dire à température constante, égale à la température T à laquelle l'enceinte du mélangeur a été chauffée, dès l'introduction desdits solides dans ledit mélangeur. Le préchauffage d'au moins l'un des deux desdits solides élémentaires pulvérulents à une température Ti telle que définie ci-dessus, préférentiellement des deux desdits solides élémentaires pulvérulents, permet le mélange à température isotherme dans ledit mélangeur. Le mélange est naturellement opéré en phase solide. Ledit mélangeur est avantageusement soumis à une agitation. D'une manière préférée, on choisit les conditions d'agitation de telle sorte qu'il n'y ait pas de cisaillements importants. Ainsi, on préfère une vitesse d'agitation lente. A titre indicatif, on peut préciser que dans le cas d'un mélangeur à socs de charrue, les conditions d'agitation varient avantageusement entre 0,2 et 1,5 m/s, en bout de pales.

Le mélangeur est avantageusement placé sous atmsophère inerte durant toute la durée de mise en oeuvre du procédé selon l'invention. En particulier, les étapes i) et ii) du procédé selon l'invention sont avantageusement mises en oeuvre sous atmosphère inerte, préférentiellement sous azote. Plus avantageusement, lesdites étapes i) et ii) sont mises en oeuvre sous atmosphère d'azote humide ou d'azote sec. L'humidification du flux d'azote est avantageusement assurée par barbotage d'azote dans l'eau. Le flux de gaz inerte, préférentiellement d'azote, est avantageusement chauffé à une température égale à T ± 10°C, la température T étant celle à laquelle l'enceinte du mélangeur est chauffée. La quantité d'eau présente dans l'azote représente par exemple de 1 à 5 % poids du poids de l'azote, de préférence de 2 à 3 % poids du poids de l'azote. Par azote sec, on entend un courant d'azote comprenant moins de 0,5 g, de préférence moins de 0,3 g d'eau par kg d'azote.

Un mode de réalisation préférée de mise en oeuvre de ladite étape ii) du procédé selon l'invention consiste à réaliser ledit mélange desdits solides sous atmosphère humide. L'humidité est par exemple assurée par un flux de vapeur sèche, un flux d'azote humide, ou apport d'eau liquide dans ledit mélangeur.

Le mélange desdits solides élémentaires aux propriétés organoleptiques conformément à ladite étape ii) du procédé selon l'invention conduit à la transformation desdits solides en ladite composition aromatique laquelle est récupérée conformément à ladite étape iii) du procédé selon l'invention. Ladite étape ii) est réalisée pendant une durée suffisante afin d'obtenir la transformation, préférentiellement la transformation complète, des solides en ladite composition aromatique. Cette durée est variable et est notamment fonction des solides élémentaires mis en oeuvre et de l'agitation. Par exemple, pour un mélange de vanilline et d'éthylvanilline, ladite étape ii) est avantageusement mise en oeuvre pendant une durée de 30 minutes à 2 heures.

Ladite composition aromatique est récupérée avant ou après refroidissement. Opéré à l'intérieur ou à l'extérieur dudit mélangeur, le refroidissement de la dite composition obtenue est effectué à une température inférieure à 40°C, de préférence inférieure à 35°C. La borne inférieure de la température de refroidissement est avantageusement la température ambiante. Ladite composition est avantageusement laissée à refroidir naturellement à l'intérieur ou à l'extérieur dudit mélangeur. Selon un mode de réalisation particulier, on laisse refroidir, au sein dudit mélangeur, la composition sous agitation et sous atmosphère inerte jusqu'à une température inférieure à 40°C, de préférence jusqu'à une température inférieure à 35°C.

Le procédé selon l'invention comprend avantageusement l'introduction d'au moins un excipient au cours de la mise en oeuvre de ladite étape ii) et/ou le mélange d'au moins un excipient avec ladite composition récupérée à la suite de ladite étape iii). Ledit excipient peut être introduit, tout ou partie, au cours de la mise en oeuvre de ladite étape ii) et/ou mélangé, tout ou partie, avec ladite composition récupérée à la suite de ladite étape iii). En d'autres termes, la quantité totale du ou des excipients peut être introduite au cours de la mise en oeuvre de ladite étape ii) du procédé selon l'invention ou bien ajoutée, par exemple par mélange à sec, à ladite composition récupérée à la suite de ladite étape iii) du procédé selon l'invention. Il est également possible de fractionner les quantités d'excipient(s) mises en oeuvre de sorte qu'une première partie du ou des excipients est introduite au cours de ladite étape ii) et que la partie restante est ajoutée, préférentiellement par mélange à sec, à ladite composition récupérée à la suite de la mise en oeuvre de ladite étape iii).

De manière préférée, la quantité totale du ou des excipient(s) est introduite au cours de la mise en oeuvre de ladite étape ii). Plus particulièrement, la quantité totale dudit ou desdits excipient(s) est introduite, préférentiellement à température ambiante, dans ledit mélangeur chauffé à ladite température T. Il est avantageux de procéder dans un premier temps au mélange desdits solides au sein dudit mélangeur puis d'y introduire le(s)dit(s) excipient(s), préférentiellement sous agitation.

Un autre mode préféré d'introduction d'au moins un excipient à ladite composition pulvérulente consiste à mélanger, au moins en partie, un ou plusieurs excipient(s) à ladite composition obtenue à la suite de ladite étape iii) du procédé selon l'invention. Ladite opération de mélange est avantageusement réalisée après refroidissement de ladite composition. Plus particulièrement, le mélange est réalisé à sec, préférentiellement à température ambiante, dans un mélangeur, par exemple un mélangeur à socs de charrue, un mélangeur à palettes ou un mélangeur à ruban(s).

Le choix du ou des excipients doit tenir compte de la destination du produit final incorporant ladite composition préparée selon le procédé de l'invention et ainsi présenter la propriété de comestibilité dès lors qu'il est mis en oeuvre dans le domaine alimentaire. La quantité d'excipient(s) peut être très variable et elle peut représenter de 0,1 à 90 % du poids du mélange final. Elle est choisie avantageusement entre 20 et 70 % en poids. On peut préciser à titre d'exemple, que l'on peut additionner de 5 à 50 % en poids d'un excipient au cours de ladite étape ii) du procédé selon l'invention puis ajouter de nouveau de 5 à 50 % en poids dudit excipient à ladite composition, préférentiellement refroidie, récupérée à la suite de ladite étape iii) du procédé selon l'invention. Il est également possible de moduler les types d'introduction selon les excipients c'est-à-dire d'introduire la quantité totale d'un excipient par exemple au cours de la mise en oeuvre de ladite étape ii) et de fractionner la quantité ajoutée d'un autre excipient ou vice versa.

Il va de soi que le même excipient peut être ajouté de façon fractionnée, c'est-à-dire au cours de la mise en oeuvre de ladite étape ii) puis à l'issue de la mise en oeuvre de ladite étape iii), ou que des excipients de nature différente peuvent être également introduits au cours de la mise en oeuvre de ladite étape ii) et/ou à l'issue de la mise en oeuvre de ladite étape iii).

On donne ci-après des exemples d'excipients susceptibles d'être utilisés dans le procédé selon l'invention qui sont donnés sans caractère limitatif. De manière plus préférée, ledit excipient est choisi parmi les corps gras, les alcools gras, les sucres, les polysaccharides, la silice, la vanilline et l'éthylvanilline.

Un premier type d'excipients sont les corps gras. Comme exemples, on peut mentionner les acides gras éventuellement sous forme de sels ou d'esters. Les acides gras mis en oeuvre sont généralement des acides gras saturés à longue chaîne, c'est-à-dire ayant une longueur de chaîne entre environ 9 et 21 atomes de carbone tels que par exemple, l'acide caprique, l'acide laurique, l'acide tridécylique, l'acide myristique, l'acide palmitique, l'acide stéarique, l'acide béhénique. Il est possible que lesdits acides soient sous forme salifiée et l'on peut citer notamment le stéarate de calcium ou de magnésium.

Comme esters d'acides gras, on peut citer en particulier le stéarate de glycéryle, le palmitate d'isopropyle, le palmitate de cétyle, le myristate d'isopropyle. On peut également citer plus spécifiquement, les esters de glycérol et d'acides gras à longue chaîne tels que le monostéarate de glycérol, le monopalmitostéarate de glycérol, le palmitostéarate de glycérol, le palmitostéarate d'éthylèneglycol, le palmitostéarate de polyglycérol, le palmitostéarate de polyglycol 1500 et 6000, le monolinoléate de glycérol ; les esters de glycérol éventuellement mono- ou diacétylés d'acides gras à longue chaîne tels que les monoglycérides monoacétylés ou diacétylés et leur mélange ; les glycérides hémisynthétiques.

On peut également ajouter un alcool gras dont la chaîne d'atomes de carbone est entre environ 16 et 22 atomes de carbone tel que par exemple, l'alcool myristylique, l'alcool palmitylique, l'alcool stéarylique. Il est également possible de mettre en oeuvre des alcools gras polyoxyéthylénés résultant de la condensation avec l'oxyde d'éthylène à raison de 6 à 20 moles d'oxyde d'éthylène par mole, d'alcools gras linéaires ou ramifiés ayant de 10 à 20 atomes de carbone tels que, par exemple, l'alcool de coprah, le tridécanol ou l'alcool myristylique.

On peut citer également parmi les corps gras, les cires telles que les cires microcristallines, la cire blanche, la cire de Carnauba, la paraffine.

Un autre type d'excipients sont les sucres. On peut citer par exemple le glucose, saccharose, fructose, galactose, ribose, maltose, sorbitol, mannitol, xylitol, lactitol, maltitol. Les sucres inversés tels que les sirops de glucose (sous forme solide) sont aussi avantageusement choisis comme excipients ainsi que les sucroglycérides dérivés d'huiles grasses telles que l'huile de coprah, l'huile de palme, l'huile de palme hydrogénée et l'huile de soja hydrogénée. Les sucroesters d'acides gras tels que le monopalmitate de saccharose, le monodistéarate de saccharose et le distéarate de saccharose sont aussi avantageusement choisis comme excipients.

Comme autres exemples d'excipients, on peut mentionner les polysaccharides, et l'on peut citer, entre autres, les produits suivants et leurs mélanges :
- les amidons dérivés notamment de blé, de maïs, d'orge, de riz, de manioc ou de pomme de terre, natifs, prégélatinisés ou modifiés et plus particulièrement les amidons natifs de maïs riches en amylose, les amidons de maïs prégélatinisés, les amidons de maïs modifiés, les amidons de maïs cireux modifiés, les amidons de maïs cireux prégélatinisés, les amidons de maïs cireux modifiés en particulier l'amidon OSSA/octénylsuccinate sodique,
- les hydrolysats d'amidon,
- les dextrines et maltodextrines résultant de l'hydrolyse d'un amidon (par exemple blé, maïs) ou d'une fécule (pomme de terre) ainsi que les β-cyclodextrines,
- la cellulose, ses éthers, notamment la méthylcellulose, l'éthylcellulose, la méthyléthylcellulose, l'hydroxypropylcellulose , ou ses esters, notamment la carboxyméthylcellulose ou la carboxyéthylcellulose éventuellement sous forme sodée,
- les gommes telles que la gomme de carraghénane, Kappa ou carraghénane Iota, la pectine, la gomme de guar, la gomme de caroube, et la gomme de xanthane, les alginates, la gomme arabique, la gomme d'acacia, l'agar-agar,

On choisit préférentiellement une maltodextrine ayant un degré d'hydrolyse mesuré par « dextrose équivalent » ou D.E inférieur à 20 et compris de préférence entre 5 et 19 et plus préférentiellement entre 6 et 15.

Comme autres excipients, on peut mentionner les farines notamment la farine de blé (native ou prégel) ; les fécules, plus particulièrement la fécule de pomme de terre, la fécule de Toloman, la fécule de maïs, la maïzena, le sagou ou le tapioca.

A titre d'excipients, il est également possible d'utiliser la gélatine (ayant de préférence, une force en gelée mesurée à l'aide d'un gélomètre de 100, 175 et 250 Bloom). Elle peut provenir indifféremment soit du traitement acide des peaux de porc et d'osséine, soit du traitement alcalin des peaux de bovins et d'osséïne.

Afin d'ajuster la puissance aromatique du mélange ou exhausser son gout, la mise en oeuvred'éthylmaltol et/ou de propénylguétol peut être envisagée. L'invention n'exclut pas l'addition d'une quantité supplémentaire de vanilline ou d'éthylvanilline.

Le choix des excipients est effectué comme mentionné précédemment en fonction de l'application envisagée.

La composition préparée selon le procédé de l'invention résulte de la transformation d'au moins des deux desdits solides élémentaires mis en oeuvredans ledit procédé en un composé défini. La composition préparée selon le procédé de l'invention est une composition aromatique, c'est-à-dire qu'elle présente des propriétés organoleptiques. Ladite composition aromatique ne comprend pas nécessairement un noyau ou un groupe aromatique au sens chimique, ses propriétés organoleptiques étant suffisantes en elles-mêmes pour utiliser ladite composition en tant qu'arôme, notamment arôme alimentaire, et/ou que parfum.

La composition préparée selon le procédé de l'invention est une composition pulvérulente, c'est-à-dire qu'elle se présente sous forme de poudre. Ladite poudre obtenue à l'issue du procédé de préparation selon l'invention est constituée de particules et/ou de granulés, les granulés ayant une taille généralement supérieure aux particules. La taille desdites particules et/ou granulés varie par exemple entre 200 µm et 10 000 µm et de préférence, entre 500 µm et 1 000 µm. Afin que la taille des particules et granulés soit compatible avec l'application envisagée, une opération de broyage peut être envisagée. Elle est conduite de telle sorte que la taille des particules exprimée par le diamètre médian (d₅₀) varie de 200 µm à 1 000 µm et se situe de préférence entre 500 µm et 800 µm. On définit le diamètre médian comme étant tel que 50 % en poids des particules ont un diamètre supérieur ou inférieur au diamètre médian. L'opération de broyage peut être effectuée dans un appareillage classique tel qu'un broyeur à palettes, un broyeur à broches, un broyeur à marteau.

La composition préparée selon le procédé de l'invention est encore caractérisée par sa température de fusion notée Tf, laquelle est différente de chacune des températures de fusion de chacun desdits solides élémentaires mis en oeuvre dans le procédé selon l'invention et inférieure à la plus basse des températures de fusion des solides mis en oeuvre dans le procédé selon l'invention.

De manière préférée, ladite composition présente une température de fusion Tf supérieure ou égale à 30°C, de préférence supérieure ou égale à 40°C.

De manière préférée, ladite composition préparée selon le procédé de l'invention est une composition cristallisée. Elle est avantageusement caractérisée par un spectre de diffraction de rayons X qui lui est propre présentant des raies pour des angles 2θ différents de ceux présentés par les raies figurant sur chacun des spectres de diffraction des rayons X des solides élémentaires dont est issue ladite composition préparée selon le procédé de l'invention.

De manière préférée, ladite composition préparée selon le procédé de l'invention est une composition comprenant essentiellement un composé à base de vanilline et d'éthylvanilline. Dans le présent texte, on entend « par composition comprenant essentiellement un composé à base de vanilline et d'éthylvanilline », une composition comprenant au moins 80 % en poids d'un mélange d'un composé vanilline/éthylvanilline de ratio molaire vanilline/éthylvanilline de 2 et de vanilline : la vanilline représentant moins de 20 % en poids dudit mélange.

Les caractéristiques dudit composé vanilline/éthylvanilline ont déjà l'objet de la demande de brevet WO 2010/046239.

Ledit composé se présente sous forme d'une poudre blanche qui a un point de fusion mesuré par analyse calorimétrique différentielle de 60 °C ± 2°C différent de celui de la vanilline et de l'éthylvanilline respectivement de 81 °C ± 1°C et de 76 °C ± 1°C. Il possède un spectre de diffraction des rayons X qui lui est spécifique et qui est différent de celui de la vanilline et de l'éthylvanilline. Le spectre dudit composé de vanilline et d'éthylvanilline révèle notamment la présence de raies aux angles 2θ (°) = 20,7 - 25,6 - 27,5 - 28,0 ; lesdites raies étant absentes sur les spectres de diffraction des rayons X de la vanilline et de l'éthylvanilline. Une autre caractéristique dudit composé réside dans sa stabilité : son spectre de diffraction des rayons X ne subit pas de modification significative au cours d'un stockage prolongé. L'évolution de son spectre a été suivie en fonction de la durée de stockage à température ambiante : sur une période de stockage prolongée (5 mois), on n'observe rigoureusement aucune modification du spectre dudit composé. On constate une absence de modification des raies spécifiques dudit composé vanilline/éthylvanilline de ratio molaire vanilline/éthylvanilline de 2. Une autre caractéristique dudit composé est qu'il s'agit d'un composé qui n'est pas ou très peu hygroscopique comme la vanilline et l'éthylvanilline. L'hygroscopicité dudit composé est déterminée en mesurant sa variation de masse après avoir été maintenu pendant 1 heure, à 40°C sous air à 80 % d'humidité relative. Ledit composé adsorbe moins de 0,5% en poids d'eau, sa teneur se situe de préférence entre 0,1 et 0,3 % en poids d'eau. Ledit composé reste parfaitement solide. Par ailleurs, ce composé présente de bonnes propriétés organoleptiques et il possède une puissance aromatique élevée, nettement supérieure à celle de la vanilline.

De manière très préférée, la composition obtenue selon le procédé de l'invention comprend au moins 80 % en poids, de préférence au moins 90 % en poids d'un mélange du composé vanilline/éthylvanilline et de vanilline. La composition obtenue comprend moins de 20 % en poids, de préférence moins de 10 % en poids d'autres phases cristallines du diagramme de phases vanilline/éthylvanilline et éventuellement de la vanilline.

Ladite composition préparée selon le procédé de l'invention et comprenant essentiellement un composé à base de vanilline et d'éthylvanilline présente des propriétés de coulabilité tel que l'indice de mottage après 24 heures de stockage à 40°C sous air à 80 % d'humidité relative sous une contrainte normale de 2 400 Pa varie entre 0,05 et 0,6.

Le procédé selon l'invention est un procédé avantageux et alternatif aux procédés déjà connus et mis en oeuvrepar la Demanderesse. En particulier, la conduite du mélange des solides élémentaires, sous forme de poudre, sous conditions isothermes, c'est-à-dire à température constante, conformément à ladite étape ii) du procédé selon l'invention permet de disposer d'un procédé simplifié dans lequel le préchauffage d'au moins l'un desdits deux solides élémentaires, de préférence desdits deux solides élémentaires, évite la mise en place d'une programmation délicate en température au sein du mélangeur. Il en résulte de nombreux avantages et en particulier une régulation en température facilitée pour maintenir constante la température du fluide caloporteur, l'absence de tout risque de fusion du mélange au cours de la mise en oeuvre de ladite étape ii), une productivité améliorée dès lors que les solides élémentaires sont introduits à la température désirée dès qu'ils sont mélangés. De plus, le procédé selon l'invention conduit à la préparation d'une composition présentant généralement directement la granulométrie désirée. En particulier, mis en oeuvre sous atmosphère d'azote sec, le procédé selon l'invention conduit à la préparation d'une composition se présentant sous la forme de particules ayant généralement directement la taille désirée sans la nécessité d'opérer un broyage.

De plus, la composition aromatique préparée selon le procédé de l'invention, en particulier la composition comprenant essentiellement un composé à base de vanilline et d'éthylvanilline, présente des propriétés de coulabilité améliorées et l'absence de mottage au stockage.

La composition préparée selon le procédé de l'invention peut être utilisée dans de nombreux domaines d'application. Elle est très avantageusement utilisée en tant qu'arôme dans le domaine de l'alimentation humaine et animale, de la pharmacie, et en tant que parfum dans l'industrie des cosmétiques, de la parfumerie et de la détergence.

Un domaine d'application privilégié de la mise en oeuvrede la composition préparée selon le procédé de l'invention est celui de la biscuiterie et pâtisserie, et plus particulièrement :
- biscuiterie sèche : biscuits sucrés de type classique, petits beurre, galettes, casse-croûte, sablés,
- pâtisserie industrielle : boudoirs champagne, langues de chat, biscuits à la cuillère, pain de gênes, génoise, madeleines, quatre-quarts, cakes, pâtisserie aux amandes, petits fours.

Les éléments fondamentaux présents dans les mélanges destinés aux industries précitées sont les protéines (gluten) et l'amidon qui sont le plus souvent apportés par la farine de froment. Pour la préparation des divers types de biscuits et gâteaux, on ajoute à la farine, des ingrédients tels que saccharose, sel, oeufs, lait, corps gras, éventuellement levures chimiques (bicarbonate de sodium ou autres levures artificielles) ou levures biologiques et farines de céréales diverses etc...

L'incorporation de la composition préparée selon le procédé de l'invention est réalisée au cours de la fabrication, en fonction du produit souhaité et est conduite selon les techniques classiques du domaine considéré (cf. notamment J.L. KIGER et J.C. KIGER - Techniques Modernes de la Biscuiterie, Pâtisserie-Boulangerie industrielles et artisanales, DUNOD, Paris, 1968, Tome 2, pp. 231 et suivantes). D'une manière préférentielle, la composition préparée selon le procédé de l'invention est introduite dans les corps gras qui interviennent dans la préparation de la pâte. A titre indicatif, on précisera que la composition préparée selon le procédé de l'invention est introduite en une quantité de 0,005 à 0,2 g par kg de pâte. La composition préparée selon le procédé de l'invention est tout à fait adaptée pour être utilisée dans le domaine de la chocolaterie et quelle que soit la forme de mise en oeuvre : chocolats en plaques, chocolats de couverture, fourrage pour chocolats. Elle peut être introduite au cours du conchage c'est-à-dire du malaxage de la pâte de cacao avec les différents ingrédients, notamment les arômes, soit après le conchage, par mise en oeuvredans le beurre de cacao. Dans ce domaine d'applications, la composition préparée selon le procédé de l'invention est utilisée selon le type de chocolat, à raison de 0,0005 g à 0,1 g pour 1 kg de produit fini : les teneurs les plus fortes se retrouvant dans le chocolat pour couverture.

Une autre utilisation de la composition préparée selon le procédé de l'invention est la fabrication des bonbons de tout genre : dragées, caramels, nougats, sucres cuits, bonbons fondants et autres.

La quantité de composition préparée selon le procédé de l'invention introduite dans les produits la contenant dépend du goût plus ou moins prononcé que l'on recherche. Ainsi, les doses d'utilisation peuvent varier entre 0,001 % et 0,2 % poids du produit dans lequel ladite composition se trouve présente. Les teneurs de ladite composition à mettre en oeuvre sont généralement faibles de l'ordre de 0,02 g pour 1 kg de produit fini.

La composition préparée selon le procédé de l'invention convient bien à des utilisations dans l'industrie laitière et plus particulièrement dans les laits aromatisés et gélifiés, les entremets, les yaourts, les glaces et les crèmes glacées.

L'aromatisation se fait par simple addition de la composition préparée selon le procédé de l'invention, dans l'un des stades de mélange requis au cours de l'élaboration du produit contenant ladite composition.

Dans le cas où la composition préparée selon le procédé de l'invrntion est une composition comprenant essentiellement un composé à base de vanilline/éthylvanilline, une autre application de la composition préparée selon le procédé de l'invention dans le domaine alimentaire est la préparation du sucre vanilliné c'est-à-dire l'imprégnation du sucre avec ladite composition, en une teneur de l'ordre de 7 g exprimée par rapport à 1 kg de produit fini.

La composition préparée selon le procédé de l'invention peut également intervenir dans différentes boissons et l'on peut citer, entre autres, la grenadine et les boissons chocolatées. En particulier, elle peut être mise en oeuvredans les préparations pour boissons instantanées délivrées par les distributeurs automatiques de boissons, boissons aromatisées en poudre, chocolat en poudre ou bien dans les préparations instantanées sous forme de poudre destinées à la confection de desserts en tout genre, flans, pâtes à gâteaux, pancakes, après dilution à l'eau ou au lait.

Il est courant d'utiliser la vanilline pour la dénaturation du beurre. A cet effet, la composition préparée selon le procédé de l'invention, lorsque ladite composition est une composition comprenant essentiellement un composé à base de vanilline/éthylvanilline, peut être mise en oeuvre à raison de 6 g par tonne de beurre.

Un autre domaine d'application de la composition préparée selon le procédé de l'invention est l'alimentation animale, notamment pour la préparation de farines pour aliments des veaux et des porcs. La teneur préconisée est d'environ 0,2 g par kg de farine à aromatiser.

La composition préparée selon le procédé de l'invention peut trouver d'autres applications comme agent de masquage, pour l'industrie pharmaceutique (masquage de l'odeur de médicament) ou pour d'autres produits industriels (de type gomme, plastique, caoutchouc...).

Elle convient tout à fait bien dans des domaines totalement différents tels que la cosmétique, l'industrie de la parfumerie ou la détergence.

Elle peut être utilisée dans les cosmétiques tels que crèmes, laits, fards et autres produits et aussi, comme ingrédients parfumants, dans les compositions parfumantes, substances et produits parfumés. Par "compositions parfumantes", on désigne des mélanges de divers ingrédients tels que solvants, supports solides ou liquides, fixateurs, composés odorants divers, etc..., dans laquelle est incorporée la composition préparée selon le procédé de l'invention, laquelle est utilisée pour procurer à divers types de produits finis, la fragrance recherchée. Les bases pour parfum constituent des exemples préférés des compositions parfumantes dans lesquelles la composition préparée selon le procédé de l'invention peut être avantageusement utilisée à raison d'une teneur de 0,1 % à 2,5 % en poids. Les bases pour parfum peuvent servir à la préparation de nombreux produits parfumés tels que, par exemple, les eaux de toilettes, les parfums, les lotions après rasage ; les produits de toilette et d'hygiène tels que les gels de bain ou de douche, les produits déodorants ou antiperspirants, qu'ils soient sous forme de sticks ou de lotions, les talcs ou poudres de toute nature ; les produits pour les cheveux tels que les shampooings et les produits capillaires de tout type. Un autre exemple de mise en oeuvre de la composition préparée selon le procédé de l'invention est le domaine de la savonnerie. Elle peut être utilisée à une teneur de 0,3 % à 0,75 % de la masse totale à parfumer. Généralement, elle est associée dans cette application, à du résinoïde de benjoin et de l'hyposulfite de sodium (2 %).

La composition préparée selon le procédé de l'invention peut trouver de nombreuses autres applications, notamment dans les désodorisants d'air ambiant ou tout produit d'entretien.

Les caractéristiques physico-chimiques des compositions préparées selon le procédé de l'invention sont déterminées selon les méthodes suivantes :
1. Point de fusion ou température de fusion.
   Le point de fusion ou température de fusion de la composition préparée selon le procédé de l'invention est mesuré par analyse calorimétrique différentielle. La mesure est effectuée à l'aide d'un analyseur différentiel Mettler DSC822e dans les conditions suivantes :
   - préparation de l'échantillon à température ambiante : pesée et introduction dans un porte échantillon,
   - porte échantillon : capsule en aluminium sertie,
   - prise d'essai : 5 à 10 mg (précisément 8,4 mg dans les exemples qui suivent),
   - vitesse de montée en température : 2-10°C/min,
   - plage d'étude : 10 - 90°C.

   On pèse l'échantillon de la composition qui est introduite dans la capsule qui est sertie puis placée dans l'appareil.
   On lance la programmation de température et l'on obtient le profil de fusion sur un thermogramme.
   La température de fusion est définie à partir d'un thermogramme réalisé dans les conditions opératoires précédentes.
   On retient la température onset : température correspondant à la pente maximale du pic de fusion.
2. Spectre de diffraction des rayons X.
   Le spectre de diffraction des rayons X de la composition préparée selon le procédé de l'invention est déterminé à l'aide de l'appareil X'Pert Pro MPD PANalytical équipé d'un détecteur X' Celerator, dans les conditions suivantes :
   - Start Position [°2Th.] : 1,5124
   - End Position [°2Th.] : 49,9794
   - Step Size [°2Th.] : 0,0170
   - Scan Step Time [s] : 41,0051
   - Anode Material : Cu
   - K-Alpha1 [Å] : 1,54060
   - Generator Settings : 30 mA, 40 kV
3. Propriété de coulabilité et indice de mottage. Cette propriété est particulièrement étudiée dans le cas d'une composition à base d'un mélange vanilline/éthylvanilline.

La composition préparée selon le procédé de l'invention présente la caractéristique de moins motter au stockage ce qui est mis en évidence par la détermination de l'indice de coulabilité de la poudre.

La coulabilité des poudres est une notion technique bien connue de l'homme du métier. Pour plus de détails, on peut se reporter notamment à l'ouvrage "Standard shear testing technique for particulate solids using the Jenike shear cell", publié par "The Institution of Chemicals Engineers", 1989 (ISBN : 0 85295 232 5).

La mesure de l'indice de coulabilité est effectuée de la manière qui suit.

La coulabilité des poudres est mesurée par cisaillement d'un échantillon dans une cellule annulaire (commercialisée par D. Schulze, Allemagne).

Le précisaillement des poudres est effectué sous une contrainte normale de 5200 Pa.

Les points de cisaillement nécessaires au tracé du lieu d'écoulement de l'échantillon sont obtenus pour 4 contraintes normales inférieures à la contrainte du précisaillement, typiquement 480 Pa, 850 Pa, 2 050 Pa et 3020 Pa.

A partir des cercles de Mohr dans le diagramme "contrainte de cisaillement en fonction des contraintes normales", on détermine sur le lieu d'écoulement deux contraintes qui caractérisent l'échantillon :
- la contrainte normale dans la direction principale ; elle est donnée par l'extrémité du grand cercle de Mohr qui passe par le point de précisaillement,
- la force de cohésion ; elle est donnée par l'extrémité du petit cercle de Mohr qui est tangent au lieu d'écoulement et passe par l'origine.

Le rapport entre la contrainte normale dans la direction principale et la force de cohésion est un nombre adimensionnel appelé "i, indice de coulabilité".

Ces mesures sont réalisées immédiatement après remplissage de la cellule annulaire, on obtient ainsi l'indice de coulabilité instantané.

Une autre série de mesures est réalisée avec une cellule qui a été stockée pendant 24 heures à 40°C et 80% d'humidité relative sous une contrainte normale de 2 400 Pa.

On obtient ainsi l'indice de mottage.

On donne ci-après des exemples illustrant la présente invention, sans caractère limitatif.

### Exemple 1 :

2100 g de vanilline (VA) en poudre et 900 g d'éthylvanilline (EVA) en poudre sont chauffés à 60°C pendant une nuit dans une étuve. Le rapport massique VA/EVA est 70/30. L'humidité de ces poudres est de 0,1 % en poids.

Un mélangeur à socs de charrue équipé d'une cuve d'un volume de 15 litres chauffée par une double enveloppe est préchauffé à 51 °C. La double enveloppe est alimentée par de l'eau thermostatée à 52°C.

Une circulation d'azote d'humide est établie dans le mélangeur avec un débit de 200 l/h. L'humidification du flux d'azote est assurée par barbotage dans de l'eau maintenue à 40°C de façon à obtenir 25 g d'eau par kg d'azote. La ligne d'alimentation entre le bain d'eau et le mélangeur est maintenue à 45°C de façon à éviter toute condensation dans les canalisations.

Après préchauffage du mélangeur à 51 °C, la vanilline préchauffée est introduite dans ledit mélangeur puis on y introduit l'éthylvanilline préchauffée. L'agitation du mélangeur est mise en service à la vitesse de 100 trs/min soit une vitesse en bout de pales de 1,25 m/s durant 5 minutes. Puis l'agitation est réduite à une vitesse de 40 trs/min (soit une vitesse de 0,5 m/s en bout de pales) et est maintenue durant 1 heure. Le chauffage de l'eau alimentant la double enveloppe est alors arrêté et par refroidisssement naturel, la température du produit est amenée jusqu'à 35°C. L'agitation du mélangeur et la circulation d'azote sont stoppées et le mélangeur est vidangé. Le produit est ensuite récupéré.

Le produit est tamisé à 1 mm ; le passant (particules ayant une taille inférieure à 1 mm) représente 56 % en poids de la masse totale. Le refus (granulés ayant une taille supérieure à 1 mm) à 1 mm est broyé à l'aide d'un broyeur Quadro Comill muni d'une grille de 1 mm. Les 2 fractions sont ensuite réunies et le mélange est homogénéisé pour donner la composition pulvérulente aux propriétés organoleptiques.

Le point de fusion de la composition obtenue est déterminé par analyse calorimétrique différentielle comme décrit précédemment. Le thermogramme obtenu présente un pic principal qui correspond au composé vanilline/éthylvanilline. La température de fusion (Tonset) qui correspond à la pente maximale du pic est de 59,5°C.

Le spectre de diffraction des rayons X de la composiiton présente les raies caractéristiques aux angles 2θ = 20,7 - 25,6 - 27,5 - 28,0 comme illustrées par la figure 1 et qui le différentient des spectres de la vanilline et de l'éthylvanilline.

L'indice de coulabilité et l'indice de mottage, mesurés comme décrit précédemment à l'aide d'une cellule annulaire, sont respectivement de 9,6 et de 0,14.

### Exemple 2 :

2000 g de la composition, obtenue dans l'exemple 1, après l'opération de tamisage sont mélangés avec 3000 g de Glucidex^{®} IT12 (maltodextrine) commercialisé par Roquette. Le mélange est réalisé à température ambiante dans un mélangeur à socs de charrue pendant une durée de 5 minutes. L'indice de coulabilité et l'indice de mottage, mesurés comme décrit précédemment à l'aide d'une cellule annulaire, sont respectivement de 15 et de 0,56. Le spectre de diffraction des rayons X de la composition ainsi obtenue en mélange avec un excipient à base de maltodextrine présente les raies caractéristiques aux angles 2θ = 20,7 - 25,6 - 27,5 - 28,0.

### Exemple 3 :

1400 g de vanilline (VA) en poudre et 600 g d'éthylvanilline (EVA) en poudre sont chauffés à 60°C pendant une nuit dans une étuve. Le rapport massique VA/EVA est 70/30. L'humidité de ces poudres est de 0,1 % en poids.

Un mélangeur à socs de charrue équipé d'une cuve d'un volume de 15 litres chauffée par une double enveloppe est préchauffé à 52°. La double enveloppe est alimentée par de l'eau thermostatée à 52°C. Le mélangeur est placé sous atmosphère inerte assurée par un balayage d'azote sec (40 l/h). Après préchauffage du mélangeur à 52°C, la vanilline préchauffée est introduite dans ledit mélangeur puis on y introduit l'éthylvanilline préchauffée. L'agitation du mélangeur est mise en service à la vitesse de 100 trs/min, soit une vitesse en bout de pales de 1,25 m/s, durant 5 minutes. Puis l'agitation est réduite à une vitesse de 40 trs/min et est maintenue durant 1 heure.

3 kg de glucidex^{®} IT12 commercialisée par Roquette (maltodextrine) sont ensuite introduits, à température ambiante, dans le mélangeur pourvu du mélange VA-EVA où se produit la transformation à 52°C pour obtenir la composition désirée. La température de la double enveloppe est maintenue à T = 52°C et on procède à une agitation du mélange à 100 trs/min pendant 5 minutes. Le chauffage de l'eau alimentant la double enveloppe est ensuite arrêté, l'agitation du mélangeur et la circulation d'azote sont stoppées. Le mélangeur est vidangé et le produit est ensuite récupéré et refroidi naturellement à l'air ambiant.

La granulométrie du produit est vérifiée par tamisage : la taille des particules (diamètre équivalent) formant le produit ainsi obtenu est inférieure à 1 mm. Aucune opération de broyage n'est nécessaire. Le point de fusion du produit obtenu est déterminé par analyse calorimétrique différentielle comme décrit précédemment. Le thermogramme obtenu présente un pic principal qui correspond au composé vanilline/éthylvanilline. La température de fusion (Tonset) qui correspond à la pente maximale du pic est de 58,5°C.

Le spectre de diffraction des rayons X des particules présente les raies caractéristiques aux angles 2θ = 20,7 - 25,6 - 27,5 - 28,0. L'indice de coulabilité et l'indice de mottage, mesurés comme décrit précédemment à l'aide d'une cellule annulaire, sont respectivement de 13 et de 0,58.

## Revendications

1. Procédé de préparation d'une composition aromatique pulvérulente présentant une température de fusion Tf comprenant :
i) l'introduction dans un mélangeur dont l'enceinte a été préalablement chauffée à une température T inférieure à Tf d'au moins deux solides élémentaires pulvérulents présentant des propriétés organoleptiques, lesdits solides étant introduits séparément dans ledit mélangeur,
ii) le mélange dans ledit mélangeur, en l'absence de toute phase liquide extérieure, desdits solides à une température T inférieure à la température Tf, au moins l'un desdits solides élémentaires pulvérulents étant introduit dans ledit mélangeur à une température Ti telle que le mélange se réalise en conditions isothermes à une température fixée à ladite température T, et
iii) la récupération de ladite composition aromatique pulvérulente.

2. Procédé de préparation selon la revendication 1 dans lequel lesdits solides élémentaires pulvérulents présentent une température de fusion supérieure ou égale à 40°C.

3. Procédé de préparation selon la revendication 1 ou la revendication 2 dans lequel lesdits solides sont choisis parmi les aldéhydes aromatiques, les cétones aromatiques, les acides aromatiques, les esters aromatiques, les éthers aromatiques, les phénols, les hétérocycles oxygénés, les hétérocycles azotés, les hétérocycles soufrés et azotés, les composés soufrés, les terpènes et les muscs.

4. Procédé de préparation selon l'une des revendications 1 à 3 dans lequel chacun desdits solides élémentaires est choisi parmi une même famille chimique choisie parmi les familles constituées par les aldéhydes aromatiques, les cétones aromatiques, les acides aromatiques, les esters aromatiques, les éthers aromatiques, les phénols, les hétérocycles oxygénés, les hétérocycles azotés, les hétérocycles soufrés et azotés, les composés soufrés, les terpènes et les muscs.

5. Procédé de préparation selon la revendication 4 dans lequel chacun desdits solides appartient à la famille des aldéhydes aromatiques

6. Procédé de préparation selon la revendication 5 dans lequel l'un des solides élémentaire est la vanilline et l'autre solide élémentaire est l'éthylvanilline.

7. Procédé de préparation selon l'une des revendications 1 à 3 dans lequel chacun desdits solides élémentaires appartient à une famille chimique différente choisie parmi les familles constituées par les aldéhydes aromatiques, les cétones aromatiques, les acides aromatiques, les esters aromatiques, les éthers aromatiques, les phénols, les hétérocycles oxygénés, les hétérocycles azotés, les hétérocycles soufrés et azotés, les composés soufrés, les terpènes et les muscs.

8. Procédé de préparation selon la revendication 7 dans lequel au moins l'un des solides élémentaires appartient à la famille des aldéhydes aromatiques, des éthers aromatiques ou des hétérocyles oxygénés.

9. Procédé de préparation selon la revendication 7 ou la revendication 8 dans lequel l'un des solides élémentaires appartient à la famille des aldéhydes aromatiques et l'autre solide élémentaires appartient à la famille des éthers aromatiques.

10. Procédé de préparation selon la revendication 7 ou la revendication 8 dans lequel l'un des solides élémentaires est choisi parmi les éthers aromatiques et l'autre solide élémentaire est choisi parmi les hétérocyles oxygénés.

11. Procédé de préparation selon la revendication 6 dans lequel l'enceinte dudit mélangeur est chauffée à une température T comprise entre 48 et 53°C.

12. Procédé de préparation selon l'une des revendication 1 à 11 dans lequel lesdits deux solides élémentaires pulvérulents sont introduits à une température Ti, ladite température Ti étant choisie telle que T - 20°C ≤ Ti ≤ T + 20°C.

13. Procédé de préparation selon la revendication 6, la revendication 11 ou la revendication 12 dans lequel la vanilline et l'éthylvanilline sont préalablement chauffées à une température comprise entre 50 et 65°C.

14. Procédé de préparation selon l'une des revendications 1 à 13 dans lequel lesdites étapes i) et ii) sont mises en oeuvre sous atmosphère d'azote humide ou d'azote sec.

15. Procédé de préparation selon l'une des revendications 1 à 14 tel qu'il comprend l'introduction d'au moins un excipient au cours de la mise en oeuvre de ladite étape ii) et/ou le mélange d'au moins un excipient avec ladite composition récupérée à la suite de ladite étape iii).

16. Procédé de préparation selon la revendication 15 tel que la quantité totale dudit ou desdits excipient(s) est introduite à température ambiante dans ledit mélangeur chauffé à ladite température T.

17. Procédé de préparation selon la revendication 15 ou la revendication 16 tel que ledit excipient est choisi parmi les corps gras, les alcools gras, les sucres, les polysaccharides, la silice, la vanilline et l'éthylvanilline.

18. Procédé de préparation selon l'une des revendications 1 à 17 tel que ladite composition aromatique présente une température de fusion Tf supérieure ou égale à 30°C.

## Patentansprüche

1. Verfahren zur Herstellung einer pulverförmigen aromatischen Zusammensetzung mit einem Schmelzpunkt Tf, bei dem man:
i) in einem Mischer, dessen Kammer auf eine Temperatur T unterhalb von Tf vorerhitzt wurde, mindestens zwei pulverförmige elementare Feststoffe mit organoleptischen Eigenschaften einträgt, wobei die Feststoffe separat in den Mischer eingetragen werden,
ii) die Feststoffe in dem Mischer in Abwesenheit von jeglicher externer flüssiger Phase bei einer Temperatur T unterhalb der Temperatur Tf mischt, wobei mindestens einer der pulverförmigen elementaren Feststoffe bei einer solchen Temperatur Ti in den Mischer eingetragen wird, dass das Mischen unter isothermen Bedingungen bei einer auf die Temperatur T fixierten Temperatur erfolgt, und
iii) die pulverförmige aromatische Zusammensetzung gewinnt.

2. Herstellungsverfahren nach Anspruch 1, bei dem die pulverförmigen elementaren Feststoffe einen Schmelzpunkt größer gleich 40°C aufweisen.

3. Herstellungsverfahren nach Anspruch 1 oder Anspruch 2, bei dem die Feststoffe aus aromatischen Aldehyden, aromatischen Ketonen, aromatischen Säuren, aromatischen Estern, aromatischen Ethern, Phenolen, sauerstoffhaltigen Heterocyclen, stickstoffhaltigen Heterocyclen, schwefel- und stickstoffhaltigen Heterocyclen, Schwefelverbindungen, Terpenen und Moschusverbindungen ausgewählt werden.

4. Herstellungsverfahren nach einem der Ansprüche 1 bis 3, bei dem jeder der elementaren Feststoffe aus derselben chemischen Familie ausgewählt wird, die aus den Familien ausgewählt wird, die aus aromatischen Aldehyden, aromatischen Ketonen, aromatischen Säuren, aromatischen Estern, aromatischen Ethern, Phenolen, sauerstoffhaltigen Heterocyclen, stickstoffhaltigen Heterocyclen, schwefel- und stickstoffhaltigen Heterocyclen, Schwefelverbindungen, Terpenen und Moschusverbindungen bestehen.

5. Herstellungsverfahren nach Anspruch 4, bei dem jeder der Feststoffe zur Familie der aromatischen Aldehyde gehört.

6. Herstellungsverfahren nach Anspruch 5, bei dem es sich bei einem der elementaren Feststoffe um Vanillin handelt und es sich bei dem anderen elementaren Feststoff um Ethylvanillin handelt.

7. Herstellungsverfahren nach einem der Ansprüche 1 bis 3, bei dem jeder der elementaren Feststoffe zu einer anderen chemischen Familie gehört, die aus den Familien ausgewählt wird, die aus aromatischen Aldehyden, aromatischen Ketonen, aromatischen Säuren, aromatischen Estern, aromatischen Ethern, Phenolen, sauerstoffhaltigen Heterocyclen, stickstoffhaltigen Heterocyclen, schwefel- und stickstoffhaltigen Heterocyclen, Schwefelverbindungen, Terpenen und Moschusverbindungen bestehen.

8. Herstellungsverfahren nach Anspruch 7, bei dem mindestens einer der elementaren Feststoffe zur Familie der aromatischen Aldehyde, aromatischen Ether oder sauerstoffhaltigen Heterocyclen gehört.

9. Herstellungsverfahren nach Anspruch 7 oder Anspruch 8, bei dem einer der elementaren Feststoffe zur Familie der aromatischen Aldehyde gehört und der andere elementare Feststoff zur Familie der aromatischen Ether gehört.

10. Herstellungsverfahren nach Anspruch 7 oder Anspruch 8, bei dem einer der elementaren Feststoffe aus den aromatischen Ether ausgewählt wird und der andere elementare Feststoff aus den sauerstoffhaltigen Heterocyclen ausgewählt wird.

11. Herstellungsverfahren nach Anspruch 6, bei dem die Kammer des Mischers auf eine Temperatur T zwischen 48 und 53°C erhitzt wird.

12. Herstellungsverfahren nach einem der Ansprüche 1 bis 11, bei dem die beiden pulverförmigen elementaren Feststoffe bei einer Temperatur Ti eingetragen werden, wobei die Temperatur Ti so gewählt wird, dass T - 20°C ≤ Ti ≤ T + 20°C.

13. Herstellungsverfahren nach Anspruch 6, Anspruch 11 oder Anspruch 12, bei dem das Vanillin und das Ethylvanillin auf eine Temperatur zwischen 50 und 65°C vorerhitzt werden.

14. Herstellungsverfahren nach einem der Ansprüche 1 bis 13, bei dem die Schritte i) und ii) unter feuchter oder trockener Stickstoffatmosphäre durchgeführt werden.

15. Herstellungsverfahren nach einem der Ansprüche 1 bis 14, bei dem im Lauf der Durchführung von Schritt ii) mindestens ein Hilfsstoff eingetragen wird und/oder mindestens ein Hilfsstoff mit der nach Schritt iii) gewonnenen Zusammensetzung gemischt wird.

16. Herstellungsverfahren nach Anspruch 15, bei dem die Gesamtmenge des Hilfsstoffs bzw. der Hilfsstoffe bei Umgebungstemperatur in den auf die Temperatur T erhitzten Mischer eingetragen wird.

17. Herstellungsverfahren nach Anspruch 15 oder Anspruch 16, bei dem der Hilfsstoff aus Fettsubstanzen, Fettalkoholen, Zuckern, Polysacchariden, Siliciumdioxid, Vanillin und Ethylvanillin ausgewählt wird.

18. Herstellungsverfahren nach einem der Ansprüche 1 bis 17, bei dem die aromatische Zusammensetzung einen Schmelzpunkt Tf größer gleich 30°C aufweist.

## Claims

1. Process for preparing a pulverulent aromatic composition having a melting point Tm, comprising:
i) the introduction into a mixer, whose chamber has been heated beforehand to a temperature T below Tm, of at least two pulverulent elementary solids with organoleptic properties, said solids being introduced separately into said mixer,
ii) the mixing in said mixer, in the absence of any external liquid phase, of said solids at a temperature T below the temperature Tm, at least one of said pulverulent elementary solids being introduced into said mixer at a temperature Ti such that the mixing takes place under isothermal conditions at a temperature set at said temperature T, and
iii) the recovery of said pulverulent aromatic composition.

2. Preparation process according to Claim 1, in which said pulverulent elementary solids have a melting point of greater than or equal to 40°C.

3. Preparation process according to Claim 1 or Claim 2, in which said solids are chosen from aromatic aldehydes, aromatic ketones, aromatic acids, aromatic esters, aromatic ethers, phenols, oxygenous heterocycles, nitrogenous heterocycles, sulfureous and nitrogenous heterocycles, sulfur compounds, terpenes and musks.

4. Preparation process according to one of Claims 1 to 3, in which each of said elementary solids is chosen from the same chemical family chosen from the families consisting of aromatic aldehydes, aromatic ketones, aromatic acids, aromatic esters, aromatic ethers, phenols, oxygenous heterocycles, nitrogenous heterocycles, sulfureous and nitrogenous heterocycles, sulfur compounds, terpenes and musks.

5. Preparation process according to Claim 4, in which each of said solids belongs to the family of aromatic aldehydes.

6. Preparation process according to Claim 5, in which one of the elementary solids is vanillin and the other elementary solid is ethylvanillin.

7. Preparation process according to one of Claims 1 to 3, in which each of said elementary solids belongs to a different chemical family chosen from the families consisting of aromatic aldehydes, aromatic ketones, aromatic acids, aromatic esters, aromatic ethers, phenols, oxygenous heterocycles, nitrogenous heterocycles, sulfureous and nitrogenous heterocycles, sulfur compounds, terpenes and musks.

8. Preparation process according to Claim 7, in which at least one of the elementary solids belongs to the family of aromatic aldehydes, aromatic ethers or oxygenous heterocycles.

9. Preparation process according to Claim 7 or Claim 8, in which one of the elementary solids belongs to the family of aromatic aldehydes and the other elementary solid belongs to the family of aromatic ethers.

10. Preparation process according to Claim 7 or Claim 8, in which one of the elementary solids is chosen from aromatic ethers and the other elementary solid is chosen from oxygenous heterocycles.

11. Preparation process according to Claim 6, in which the chamber of said mixer is heated to a temperature T of between 48 and 53°C.

12. Preparation process according to one of Claims 1 to 11, in which said two pulverulent elementary solids are introduced at a temperature Ti, said temperature Ti being chosen such that T - 20°C ≤ Ti ≤ T + 20°C.

13. Preparation process according to Claim 6, Claim 11 or Claim 12, in which the vanillin and the ethylvanillin are preheated to a temperature of between 50 and 65°C.

14. Preparation process according to one of Claims 1 to 13, in which said steps i) and ii) are performed under an atmosphere of wet nitrogen or of dry nitrogen.

15. Preparation process according to one of Claims 1 to 14, such that it comprises the introduction of at least one excipient during the implementation of said step ii) and/or the mixing of at least one excipient with said composition recovered after said step iii).

16. Preparation process according to Claim 15, such that the total amount of said excipient(s) is introduced at room temperature into said mixer heated to said temperature T.

17. Preparation process according to Claim 15 or Claim 16, such that said excipient is chosen from fatty substances, fatty alcohols, sugars, polysaccharides, silica, vanillin and ethylvanillin.

18. Preparation process according to one of Claims 1 to 17, such that said aromatic composition has a melting point Tm of greater than or equal to 30°C.
